(19) Europäisches Patentamt
European Patent Office
Office européen des brevets

(11) Veröffentlichungsnummer: **0 118 822**
**B1**

(12) **EUROPÄISCHE PATENTSCHRIFT**

(45) Veröffentlichungstag der Patentschrift: **09.04.86**

(51) Int. Cl.⁴: **C 07 C 153/05**, A 01 N 37/00, C 07 F 7/18

(21) Anmeldenummer: **84102019.1**

(22) Anmeldetag: **27.02.84**

(54) **Substituierte Hydroxy-malonsäureamidthioamide, Verfahren zu ihrer Herstellung sowie ihre Verwendung als Schädlingsbekämpfungsmittel.**

(30) Priorität: **10.03.83 DE 3308464**

(43) Veröffentlichungstag der Anmeldung: **19.09.84 Patentblatt 84/38**

(45) Bekanntmachung des Hinweises auf die Patenterteilung: **09.04.86 Patentblatt 86/15**

(84) Benannte Vertragsstaaten: **AT BE CH DE FR GB IT LI NL**

(56) Entgegenhaltungen:
**EP-A-0 076 957**
**DE-A-2 533 716**

**HELVETICA CHIMICA ACTA, Band 51, Nr. 2, März 1968, Seiten 325-339 J. KISS et al.: "Synthese und Eigenschaften von 2-(alpha-Hydroxyalkyl)- und 2-(alpha-alkoxycarbonyl)-substituierten 4-Methyl-5-(beta-hydroxyäthyl)-thiazolen und -thiazoliumsalzen"**

(73) Patentinhaber: **BAYER AG, Konzernverwaltung RP Patentabteilung, D-5090 Leverkusen 1 Bayerwerk (DE)**

(72) Erfinder: **Fauss, Rudolf, Dr., Gerstenkamp 10, D-5000 Koeln 80 (DE)**
Erfinder: **Findeisen, Kurt, Dr., In der Follmühle 10, D-5068 Odenthal 2 (DE)**
Erfinder: **Becker, Benedikt, Dr., Metzkausenerstrasse 14, D-4020 Mettmann (DE)**
Erfinder: **Hammann, Ingeborg, Dr., Lutherstrasse 22, D-4330 Mühlheim/Ruhr (DE)**
Erfinder: **Homeyer, Bernhard, Dr., Obere Strasse 28, D-5090 Leverkusen 3 (DE)**

**EP 0 118 822 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue substituierte Hydroxy-malonsäureamidthioamide, ein Verfahren zu ihrer Herstellung sowie ihre Verwendung als Schädlingsbekämpfungsmittel.

Es ist bereits bekannt geworden, daß Carbamate wie 5,6-Dimethyl-2-dimethylamino-4-pyrimidinyl-dimethylcarbamat oder 1-Naphthyl-H-methylcarbamat insektizide Wirksamkeit besitzen (US-PS 3 493 574, 2 903 478). Ihre Wirkung ist jedoch vor allem bei niedrigen Aufwandkonzentrationen nicht immer voll befriedigend.

Aus der DE-OS 2 533 716 ist die Wirkung von N-Alkyloder Cycloalkylthioacrylamiden als Schädlingsbekämpfungsmittel bekannt. Die dort beschriebenen Verbindungen unterscheiden sich jedoch strukturell wesentlich von den erfindungsgemäßen Hydroxymalonsäureamidthioamiden.

Es wurden die neuen substituierten Hydroxymalonsäureamidthioamide der Formel (I) gefunden

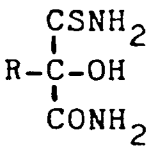

(I)

in welcher

R für Phenyl steht, das gegebenenfalls gleich oder verschieden durch einen oder mehrere der folgenden Reste substituiert ist: Halogen, Nitro, Amino, OH, CN, $C_{1-4}$-Alkyl, $C_{1-4}$-Halogenalkyl, $C_{1-4}$-Alkoxy, $C_{1-4}$-Halogen-alkoxy, Methylendioxy, Ethylendioxy, Difluormethylendioxy, halogensubstituiertes Ethylendioxy, $C_{1-4}$-Alkylthio, $C_{1-4}$-Halogenalkylthio, $C_{2-8}$-Alkoxyalkyl, $C_{2-8}$Halogen-alkoxyalkyl, $C_{1-4}$-Alkylsulfonyl, $C_{1-4}$-Halogenalkylsulfonyl, Carboxyl, Carbalkoxy.

Weiter wurde gefunden, daß man die substituierten Hydroxymalonsäureamidthioamide der Formel (I) erhält, wobei der Rest R die oben angegebene Bedeutung besitzt, wenn man Hydroxy- bzw. Trimethylsilyloxymalonsäurethioamidnitrile der allgemeinen Formel (II)

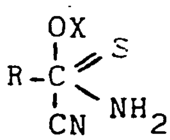

(II)

worin

R die oben angegebene Redeutung hat und

X für Wasserstoff oder Trimethylsilyl steht, mit anorganischen Säuren verseift.

Überraschenderweise zeigen die erfindungsgemäßen substituierten Hydroxy-malonsäureamidthioamide eine erheblich höhere insektizide Wirkung als die aus dem Stand der Technik bekannten Carbamate der gleichen Wirkungsrichtung. Die erfindungsgemäßen Stoffe eignen sich somit zur Schädlingsbekämpfung.

Bevorzugt sind substituierte Hydroxymalonsäureamidthioamide der Formel (I), in welcher

R für Phenyl steht, das gegebenenfalls durch Halogen, $C_{1-4}$-Alkyl, $C_{1-4}$-Alkoxy, $C_{1-4}$-Halogenalkoxy, $C_{1-4}$-Halogenalkyl, $NH_2$ oder Nitro substituiert ist.

Insbesondere bevorzugt sind substituierte Hydroxymalonsäureamidthioamide der Formel (I), in welcher

R für Phenyl steht, das gegebenenfalls ein- oder mehrfach durch Chlor oder Fluor substituiert ist.

Als neue Wirkstoffe seien im einzelnen genannt:

Phenyl-hydroxy-malonsäureamidthioamid, o-, m-, p-Chlorophenyl-hydroxy-malonsäureamidthioamid, 2,3-Dichlorphenyl-hydroxy-malonsäureamidthioamid, 3,4-Dichlorophenyl-hydroxymalonsäureamidthioamid, 3,5-Dichlorophenyl-hydroxy-malonsäureamidthioamid, 2,4-Dichlorophenyl-hydroxy-malonsäureamidthioamid, 2,5-Dichlorphenyl-hydroxy-malonsäureamidthioamid, 2,6-Dichlorophenyl-hydroxy-malonsäureamidthioamid, 3,4,5 Trichlorphenyl-hydroxymalonsäureamid-thioamid, o-, m-, p-Nitrophenyl-hydroxy-malonsäure amidthioamid, o-Chloro-methylphenyl-hydroxy-malonsäureamidthioamid, o-, m-, p-Trifluormethylphenyl-hydroxy-malonsäureamidthioamid, o-, m-, p-Methoxyphenylhydroxy-malonsäureamidthioamid, 2,6-Dimethoxyphenyl-hydroxy-malonsäureamidthioamid, o-, m-, p-Tolylhydroxymalonsäureamidthioamid, o-, m-, p-Trifluoromethoxyphenylhydroxymalonsäureamidthioamid, o-, m-, p-Fluorphenylhydroxymalonsäureamidthioamid.

Verwendet man Trimethylsilyloxyphenylmalonsäurethioamidnitril als Ausgangsstoff und 96 %ige Schwefelsäure als Verseifungsmittel, so kann der Reaktionsablauf durch das folgende Formelschema wiedergegeben werden:

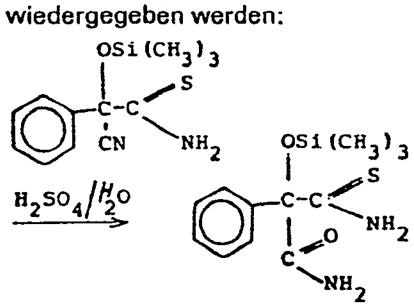


Als Verdünnungsmittel bei der Verseifung verwendet man im allgemeinen Wasser. Die Verseifung kann in wasserfreien- oder Wasser enthaltenden Säuren durchgeführt werden, häufig genügen schon geringe Mengen Wasser, um die gewünschten Endprodukte zu erhalten. Die Wassermenge kann aber in einem größeren

Bereich, zwischen 2 % und 75 % bezogen auf die eingesetzte Säuremenge, variiert werden. Es ist weiterhin möglich, die Ausgangsprodukte der Formel II zuerst in einer wasserfreien Säure zu lösen und die benötigte Wassermenge zu einem späteren Zeitpunkt hinzuzufügen.

Als geeignete Säuren seien vorzugsweise genannt: Schwefelsäure, Phosphorsäure, Salzsäure, Flußsäure, Perchlorsäure, Borsäure. Besonders bevorzugt sind Schwefelsäure ($H_2SO_4$) und Salzsäure (HCl).

In einigen Fällen sind die Endprodukte der allgemeinen Formel I etwas wasserlöslich und müssen deshalb mit Hilfe eines Extraktionsmittels aus diesem entfernt werden. Als Extraktionsmittel kommen alle inerten organischen Lösungsmittel, die sich mit Wasser nicht, oder nur wenig, mischen, in Betracht. Hierzu gehören Toluol, Xylol, Chlorbenzol, Dichlorobenzol, Essigsäureethylester, Methylenchlorid, Chloroform, Tetrachlorkohlenstoff und Ether. Im allgemeinen ist ein Extraktionsmittel aber nicht nötig.

Die Reaktionstemperaturen können in einem größeren Bereich variiert werden. Im allgemeinen arbeitet man zwischen etwa -15°C bis 100°C, vorzugsweise zwischen 0 und 80°C, insbesondere zwischen 20 und 60°C.

Die Umsetzung wird normalerweise bei Normaldruck. Sie kann aber auch bei erhöhtem Druck durchgeführt werden. Im allgemeinen arbeitet man bei Drücken zwischen etwa 1 bar und etwa 10 bar, vorzugsweise zwischen 1 bar und bar.

Bei der Durchführung des erfindungsgemäßen Verfahrens setzt man auf 1 Mol der Hydroxy- bzw. Trimethylsilyloxy-malonsäurethioamidnitrile (Formel II) etwa 0,5 bis 20 Mol, vorzugsweise 1 bis 5 Mol an anorganischer Säure ein.

Die in der Säure gelöste Verbindungen der Formel II wird nach 30 Minuten bis zwei Stunden Rühren auf Eis gegossen und entweder durch Absaugen oder Extraktion gewonnen. Die Reinigung wird im allgemeinen durch Umkristallisation vorgenommen.

Verbindungen der Formel II sind neu.

Sie werden erhalten, indem man

1) α-Ketosäurethioamide der allgemeinen Formel III

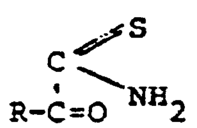

(III)

wobei

R die oben angegebene Bedeutung hat mit HCN oder HCN-abspaltenden Mitteln oder Trimethylsilylcyanid umsetzt, oder

2) Trimethylsilyloxymalonsäuredinitrile der allgemeinen Formel IV

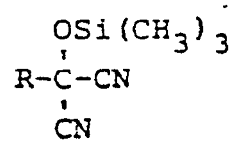

(IV)

worin

R die oben angegebene Bedeutung hat mit $H_2S$ umsetzt.

Verfahren 1 sei durch folgende 3 Reaktionsgleichungen illustriert:

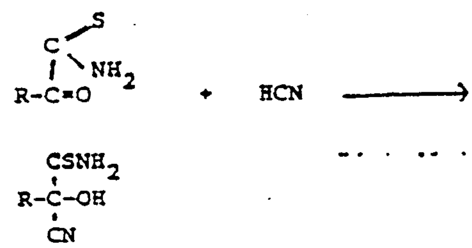

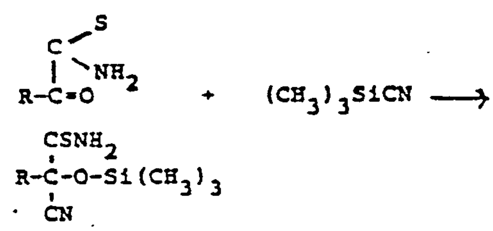

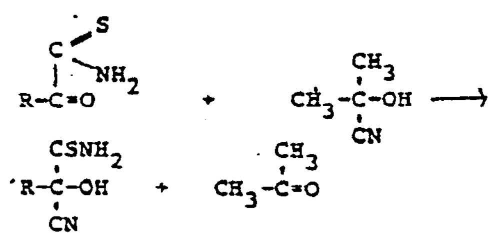

Als HCN-abspaltende Substanzen kommen Cyanhydrine zum Einsatz, beispielsweise seien Acetoncyanhydrin oder Benzaldehydcyanhydrin genannt.

Verwendet man Phenylglyoxylsäurethioamid und Trimethylsilylcyanid als Ausgangsprodukte für die Umsetzung, so kann der Reaktionsablauf durch das folgende Formelschema wiedergegeben werden:

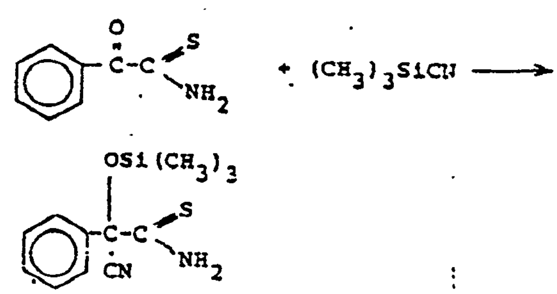

Die Umsetzung von III zu II verläuft nach der in der Literatur bekannten Methoden unter Anwesenheit geringer Mengen alkalischer

Katalysatoren, beispielsweise Natriumcyanid, Kaliumcyanid oder tertiäre Amine wie Triethylamin, sowie Alkali- und Erdalkalihydroxide und -carbonate.

Während die Addition von molaren Mengen HCN bzw. Trimethylsilylcyanid leicht exothern bereits bei Raumtemperatur verläuft und nach beendeter Zugabe der Reaganzien abgeschlossen ist muß bei Einsatz von Cyanhydrinen einige Zeit erhitzt werden um einen vollständigen Umsatz zu erreichen. Hierbei ist es auch sinnvoll auf 1 Mol α - Ketothioamid der Formel III mehr als 1 Mol Cyanhydrin anzusetzen. Bevorzugt werden 1,1 - 3 bevorzugt 1,2 - 2 Mol Cyanhydrin verwendet. Der Überschuß wird nach beendeter Reaktion wieder abdestilliert.

Die Reaktionen verlaufen im allgemeinen ohne Anwesenheit von Lösungsmittel, jedoch kann die Verwendung von gegenüber den Produkten und Edukten inerten Lösungsmitteln wie z.B. Methanol, Ethanol, Isopropanol, Toluol, Chlorbenzol, Methylenchlorid, Tetrachlorkohlenstoff und Diethylether in einigen Fällen durchaus sinnvoll sein.

Die Reaktionsbedingungen für die Umsetzungen sind sehr variabel. So können z.B. HCN und Trimethylsilylcyanid auch überstöchiometrisch eingesetzt werden, jedoch bringt ein mehr als 10 %iger molarer Überschuß keinerlei Vorteile mehr. Ebenso ist die Reaktionstemperatur in weiten Grenzen variierbar, es kann im Temperaturbereich von -50 bis 300°C gearbeitet werden, wobei bei höheren Temperaturen entweder in der Gasphase oder in der Flüssigphase unter Druck die Umsetzungen vorgenommen werden. Aber auch hier ergibt sich gegenüber den zuerst beschriebenen bevorzugten Versuchsparametern kein signifikanter Vorteil.

Die Addition des Trimethylsilylcyanids an α-Ketothioamide ist überraschend und nicht direkt vorhersehbar. Aufgrund des hohen Silylierungspotentials des Trimethylsilylcyanids wäre eher eine N-Silylierung unter Blausäureabspaltung zu erwarten gewesen.

Verbindungen der allgemeinen Formel III sind teilweise bekannt und lassen sich nach bekannten Verfahren leicht herstellen (Ann. 691, 92 (1966)).

Verwendet man für das Verfahren 2 3,4-Dichlorphenyltrimethylsilyloxymalonsäuredinitril als Ausgangsprodukt für die Umsetzung mit $H_2S$, so kann der Reaktionsverlauf durch folgendes Formelschema wiedergegeben werden:

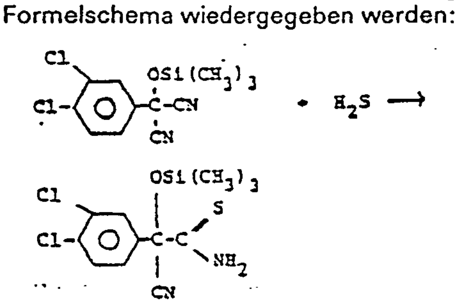

Die Umsetzung verläuft nach der in der Literatur bekannten Verfahrensvariante zur Herstellung von α-Ketothioamiden III.

Hierbei muß als überraschend angesehen werden, daß selektiv nur eine der beiden Nitrilgruppen der Verbindung IV von $H_2S$ angegriffen wird.

Im allgemeinen wird die Ausgangsverbindung IV in inerten organischen Lösungsmitteln gelöst und in Anwesenheit von alkalischen Katalysatoren mit Schwefelwasserstoff umgesetzt. Als Lösungsmittel kommen beispielsweise Toluol, Xylol, Chlorbenzol, Methylenchlorid zur Anwendung. Als Katalysatoren werden die im Verfahren 1 genannten Verbindungen eingesetzt, bevorzugt sind die tert. aliphatischen Amine, beispielsweise Triethylamin. Sie werden in Mengen von 0,01 - 5 %, bevorzugt 0,1 - 2 % bezogen auf die Verbindung IV eingesetzt.

Die Reaktion wird bei Temperaturen von -30 bis 40°C bevorzugt -10 bis 30°C durchgeführt.

Im allgemeinen wird das Trimethylsilyloxymalonsäuredinitril mit dem Katalysator im Lösungsmittel bei ca. 0°C mit $H_2S$ versetzt. Hierbei muß mindestens die der Stöchiometrie entsprechende Menge an $H_2S$ angewendet werden, vorteilhaft jedoch ein Überschuß. Anschließend rührt man bei RaumtemPeratur nach, wobei das Endprodukt bereits ausfallen kann und nur noch abgesaugt werden muß. Anderenfalls wird eingeengt und dann umkristallisiert.

Trimethylsilyloxymalonsäuredinitrile der allgemeinen Formel IV sind zum Teil bekannt (Chem. Ber. 106, 587 (1973), Tetrahedron Letters Nr. 17, 1449-1450 (1973)).

Neue Verbindungen können nach den dort angegebenen Methoden leicht entsprechend dem folgenden Formelschema synthetisiert werden

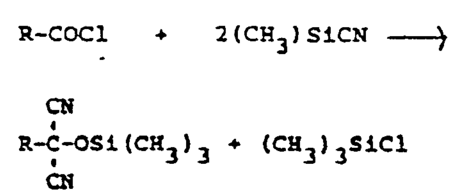

wobei R die vorgenannte Bedeutung besitzt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Calleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, HylotruPes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Cibbium psylloides, Tribolium spp., Tenebrio molitor, Agriotes spp., Conoderus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Castrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie

Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Ei-weißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

**Beispiel 1**

3,4-Dichlorphenyltrimethylsilyloxymalonsäurethioamidnitril

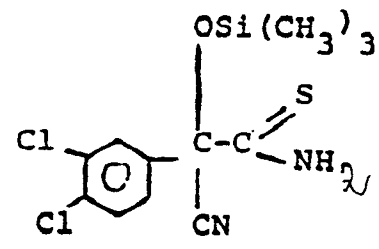

**Verfahren 2**

120 g 3,4-Dichlorphenyltrimethylsilyloxymalonsäuredinitril werden in 600 ml absolutem Toluol mit 2 ml Triethylamin versetzt und anschließend bei −5°C trockenes $H_2S$ eingeleitet. Es fielen dabei Kristalle aus. Nachdem auf Raumtemperatur erwärmt worden war wurde abgesaugt und mit etwas Toluol gewaschen. Durch Umkristallisieren aus 700 ml Toluol wurden 100 g 3,4-Dichlorphenyltrimethylsilyloxymalonsäurethioamidnitril erhalten. Die Substanz schmolz bei 100-128°C und war nicht vollständig vom Toluol zu befreien.

**Verfahren 1**

23,4 g 3,4-Dichlorphenylglyoxylsäurethioamid wurde in 150 ml absolutem Methylenchlorid suspendiert, mit 0,2 ml Triethylamin und anschließend bei Raumtemperatur langsam mit 10 g Trimethylsilylcyanid versetzt.

Es entstand eine homogene Lösung. Das Lösungsmittel wurde eingeengt und der Rückstand aus Toluol umkristallisiert. Es blieben 14 g 3,4-Dichlorphenyl-trimethylsilyloxymalonsäurethioamidnitril. Die Substanz schmolz bei 110-116°C und war nicht vollständig vom Toluol zu befreien.

**Beispiel 2**

3,4-Dichlorphenylhydroxymalonsäureamidthioamid

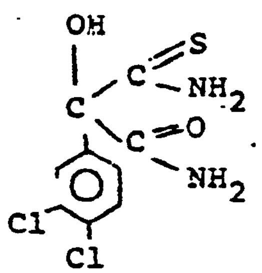

In 300 ml Schwefelsäure wurde bei max. 30°C 66,6 g 3,4-Dichlorphenyltrimethylsilyloxymalonsäurethioamidnitril eingetragen. Nach einer Stunde wurde auf Eiswasser gegeben, der Rückstand abgesaugt, in $CH_2Cl_2$ aufgenommen, mit $NaHCO_3$-Lösung und Wasser ausgeschüttelt und dann die organische Phase mit Aktivkohle ausgerührt und aufgearbeitet.

Es blieben 47,5 g eines hochviskosen Rückstandes der nach spektroskopischen Untersuchungen zum überwiegenden Teil aus gewünschtem Endprodukt bestand.

Eine kleine Menge wurde über Kieselgel filtriert (Kieselgel 60, Firma Merck; Laufmittel: Toluol/Essigsäureethylester 1:1). Das saubere 3,4-Dichlorphenylhydroxymalonsäureamidthionamid kristallisierte aus konzentrierter Chloroformlösung aus. Fp. 104-105°C.

**Beispiel 3**

Analog zu Beispiel 1 Verfahren 2 wurde Phenyltrimethylsilyloxymalonsäurethioamidnitril vom Fp. 143°C (Toluol) hergestellt. Ausbeute: 80 %.

**Beispiel 4**

Phenylhydroxymalonsäurethioamidnitril

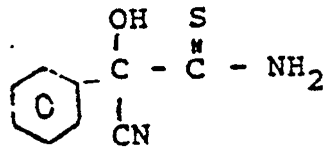

33 g Phenylglyoxylsäurethioamid wurden in 250 ml Methylenchlorid suspendiert, 0,2 ml Triethylamin zugegeben und bei 16°C eine Lösung von 5,5 g Blausäure in 15 ml Methylenchlorid zugetropft. Die Lösung wurde homogen, dann fiel das Endprodukt aus. Nach 1 Stunde wurde abgesaugt mit wenig Methylenchlorid, welches eine Spur Toluolsulfonsäure enthielt, nachgewaschen. Es blieben 22 g Phenylhydroxymalonsäurethioamidnitril; Fp. 110°C (Zersetzung).

**Beispiel 5**

Phenylhydroxymalonsäureamidthioamid

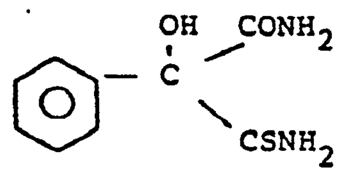

In 130 ml $H_2SO_4$ wurde bei maximal 35°C 22 g Phenylhydroxymalonsäurethioamidnitril eingetragen. Nach 1 Stunde wurde die dunkelrote Lösung auf Eis gegossen, der Niederschlag abgesaugt und gut gewaschen. Zur weiteren Reinigung wurde wie bei Beispiel 2 verfahren. Auch hier konnte des Endprodukt ohne Auftrennung über eine Kieselgelsäure nicht kristallin erhalten werden. Die spektroskopischen Daten (IR, NMR, MS (DCI)) bestätigten aber die Existenz.

**Beispiel A**

Plutella-Test

Lösungsmittel: 3 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen von Plutella maeulipennis besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 2.

**Beispiel B**

Phaedon-Larven-Test

Lösungsmittel: 3 Gewichtsteile Dimethyltormamide

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 2.

**Beispiel C**

Tetranychus-Test (resistent)

Lösungsmittel: 3 Gewichtsteile Dimethylformamide

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranyhus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2.

**Beispiel D**

Grenzkonzentrations-Test / Bodeninsekten

Testinsekt: Phorbia antiqua (im Boden)

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 5.

**Patentansprüche**

1. Substituierte Hydroxymalonsäureamidthioamide der Formel (I)

$$\begin{array}{c} CSNH_2 \\ | \\ R-C-OH \\ | \\ CONH_2 \end{array}$$

in welcher

R für Phenyl steht, das gegebenenfalls gleich

oder verschieden durch einen oder mehrere der folgende Reste substituiert ist: Halogen, Nitro, Amino, OH, CN, $C_{1-4}$-Alkyl, $C_{1-4}$-Halogenalkyl, $C_{1-4}$-Alkoxy, $C_{1-4}$-Halogen-alkoxy, Methylendioxy, Ethylendioxy, Difluormethylendioxy, halogensubstituiertes Ethylendioxy, $C_{1-4}$-Alkylthio, $C_{1-4}$-Halogenalkylthio, $C_{2-8}$-Alkoxyalkyl, $C_{2-8}$-Halogenalkoxyalkyl, $C_{1-4}$-Alkylsulfonyl, $C_{1-4}$-Halogenalkylsulfonyl, Carboxyl, Carbalkoxy.

2. Substituierte Hydroxymalonsäureamidthiamide der Formel (I) gemäß Anspruch 1, in welcher
R für Phenyl steht, das gegebenenfalls durch Halogen, $C_{1-4}$-Alkyl, $C_{1-4}$-Alkoxy, $C_{1-4}$-Halogenalkoxy, $C_{1-4}$-Halogenalkyl, $NH_2$ oder Nitro substituiert ist.

3. Substituierte Hydroxymalonsäureamidthioamide der Formel (I) gemäß Anspruch 1, in welcher
R für Phenyl steht, das gegebenenfalls ein- oder mehrfach durch Chlor oder Fluor substituiert ist.

4. Verfahren zur Herstellung der substituierten Hydroxymalonsäureamidthiamide der Formel (I)

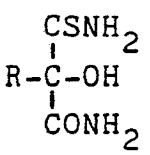

(I)

in welcher
R für Phenyl steht, das gegebenenfalls gleich oder verschieden durch einen oder mehrere der folgenden Reste substituiert ist: Halogen, Nitro, Amino, OH, CN, $C_{1-4}$-Alkyl, $C_{1-4}$-Halogenalkyl, $C_{1-4}$-Alkoxy, $C_{1-4}$-Halogen-alkoxy, Methylendioxy, Ethylendioxy, Difluormethylendioxy, halogensubstituiertes Ethylendioxy, $C_{1-4}$-Alkylthio, $C_{1-4}$-Halogenalkylthio, $C_{2-8}$-Alkoxyalkyl, $C_{2-8}$Halogen-alkoxyalkyl, $C_{1-4}$-Alkylsulfonyl, $C_{1-4}$-Halogenalkylsulfonyl, Carboxyl, Carbalkoxy,
dadurch gekennzeichnet, daß man Hydroxy- bzw. Trimethylsilyloxymalonsäurethioamidnitrile der allgemeinen Formel (II)

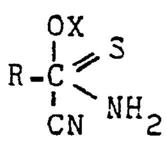

(II)

worin
R die oben angegebene Bedeutung hat und
X für Wasserstoff oder Trimethylsilyl steht, mit anorganischen Säuren verseift.

5. Schädlingsbekämpfungsmittel, gekennzeichnet durch einen Gehalt an mindestens einem substituierten Hydroxymalonsäureamidthioamid der Formel (I) gemäß Anspruch

6. Verwendung von substituierten Hydroxymalonsäureamidthioamiden der Formel (I) gemäß Anspruch 1 zur Bekämpfung von Schädlingen.

7. Verfahren zur Herstellung von Schädlingsbekämpfungsmitteln, dadurch gekennzeichnet, daß man substituierte Hydroxymalonsäureamidthioamide der Formel (I) gemäß Anspruch 1 mit Streckmitteln und/oder oberflächenaktiven Mitteln vermischt.

**Claims**

Substituted hydroxymalonic acid amide-thioamides of the formula (I)

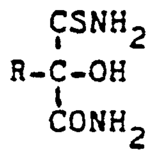

in which
R represents phenyl, which is optionally substituted by one or more identical or different radicals from the following groups: halogen, nitro, amino OH CN $C_{1-4}$-alkyl $C_{1-4}$-halogenoalkyl, $C_{1-4}$-alkoxy, $C_{1-4}$-halogenoalkoxy, methylenedioxy, ethylenedioxy, difluoromethylenedioxy, halogen-substituted ethylenedioxy, $C_{1-4}$-alkylthio, $C_{1-4}$-halogenalkylthio, $C_{2-8}$-alkoxyalkyl, $C_{2-8}$-halogenoalkoxyalkyl, $C_{1-4}$-alkylsulphonyl, $C_{1-4}$-halogenoalkylsulphonyl, carboxyl and carbalkoxy.

2. Substituted hydroxysalonic acid amide-thioamides
of the formula (I) according to Claim 1, in which R represents phenyl, which is optionally substituted by halogen $C_{1-4}$-alkyl $C_{1-4}$-alkoxy $C_{1-4}$-halogenoalkoxy, $C_{1-4}$-halogenoalkyl, $NH_2$ or nitro.

3. Substituted hydroxymalonic acid amide-thioamides of the formula (I) according to Claim 1, in which R represents phenyl, which is optionally monosubstituted or polysubstituted by chlorine or fluorine.

4. Process for the preparation of the substituted hydroxy-malonic acid amide-thioamides of the formula (I)

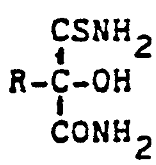

(I)

in which
R represents phenyl, which is optionally substituted by one or more identical or different radicals from the following groups: halogen, nitro, amino OH CN $C_{1-4}$-alkyl $C_{1-4}$-halogenoalkyl, $C_{1-4}$-alkoxy, $C_{1-4}$-halogenoalkoxy, methylenedioxy, ethylenedioxy, difluoromethylenedioxy, alogen-substituted ethylenedioxy, $C_{1-4}$-alkylthio, $C_{1-4}$-halogenoalkylthio $C_{2-8}$-alkoxyalkyl, $C_{2-8}$-halogenoalkoxyalkyl, $C_{1-4}$-alkylsulphonyl, $C_{1-4}$-

halogenoalkylsulphonyl, carboxyl and carbalkoxy, characterised in that hydroxy- or trimethylsilyloxymalonic acid thioamide-nitriles of the general formula (II)

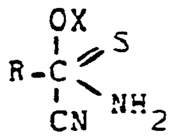

(II)

in which
R has the abovementioned meaning and
X represents hydrogen or trimethylsilyl, are hydrolysed with inorganic acids.

5. Agents for combating pests, characterised in that they contain at least one substituted hydroxymalonic acid amide-thioamide of the formula (I) according to Claim 1.

6. Use of substituted hydroxymalonic acid amidethioamides of the formula (I) according to Claim 1 for combating pests.

7. Process for the preparation of agents for combating pests, characterised in that substituted hydroxymalonic acid amide-thioamides of the formula (I) according to Claim 1 are mixed with extenders and/or surface-active agents.

**Revendications**

1. Amido-thioamides d'acides hydroxymaloniques substitués de formule (I)

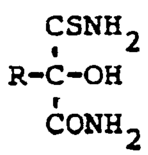

dans laquelle
R est un groupe phényle qui porte éventuellement un ou plusieurs substituants identiques ou différents choisis entre les restes suivants: halogéno, nitro, amino, OH, CN, alkyle en $C_1$ à $C_4$, halogénalkyle en $C_1$ à $C_4$, alkoxy en $C_1$ à $C_4$, halogénalkoxy en $C_1$ à $C_4$, méthylènedioxy, éthylènedioxy, difluorométhylènedioxy, éthylènedioxy à substituant halogéno, alkylthio en $C_1$ à $C_4$, halogénalkylthio en $C_1$ à $C_4$, alkoxyalkyle en $C_2$ à $C_8$, halogénalkoxyalkyle en $C_2$ à $C_8$, alkylsulfonyle en $C_1$ à $C_4$, halogénalkylsulfonyle en $C_1$ à $C_4$, carboxyle, carbalkoxy.

2. Amido-thioamides d'acides hydroxymaloniques substitués de formule (I) suivant la revendication 1, dans lesquels
R représente un groupe phényle qui est éventuellement substitué par un substituant halogéno, alkyle en $C_1$ à $C_4$, alkoxy en $C_1$ à $C_4$, halogénalkoxy en $C_1$ à $C_4$, halogénalkyle en $C_1$ à $C_4$, $NH_2$ ou nitro.

3. Amido-thioamides d'acides hydroxymaloniques substitués de formule (I) suivant la revendication 1, dans lesquels
R est un groupe phényle qui est éventuellement substitué une ou plusieurs fois par du chlore ou du fluor.

4. Procédé de production des amido-thioamides d'acides hydroxymaloniquea substitués de formule (I)

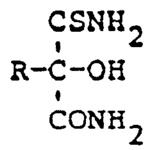

(I)

dans laquelle
R est un groupe phényle qui porte éventuellement un ou plusieurs substituants identiques ou différents choisis entre les restes suivants: halogéno, nitro, amino, OH, CN, alkyle en $C_1$ à $C_4$, halogénalkyle en $C_1$ à $C_4$, alkoxy en $C_1$ à $C_4$, halogénalkoxy en $C_1$ à $C_4$, méthylènedioxy, éthylènedioxy, difluorométhylènedioxy, éthylènedioxy à substituant halogéno, alkylthio en $C_1$ à $C_4$, halogénalkyl; thio en $C_1$ à $C_4$, alkoxyalkyle en $C_2$ à $C_8$, halogénalkoxyalkyle en $C_2$ à $C_8$, alkylsulfonyle en $C_1$ à $C_4$, halogénalkylsulfonyle en $C_1$ à $C_4$, carboxyle, carbalkoxy, caractérisé en ce qu'on saponifie avec des acides inorganiques des thioamidonitriles d'acide hydroxy- ou triméthylsilyloxymalonique de formule générale (II)

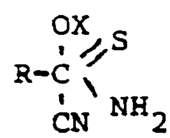

(II)

dans laquelle
R a la définition indiquée ci-dessus et
X représente l'hydrogène ou le groupe triméthylsilyle.

5. Compositions pesticides, caractérisées par une teneur en au moins un amido-thioamide d'acide hydroxymalonique substitué de formule (I) suivant la revendication 1.

6. Utilisation d'amido-thioamides d'acides hydroxymaloniques substitués de formule (I) suivant la revendication 1 pour combattre des parasites.

7. Procédé de préparation de compositions pesticides, caractérisé en ce qu'on mélange des amidothioamides d'acides hydroxymaloniques substitués de formule (I) suivant la revendication 1 avec des diluants et/ou des agents tensio-actifs.